(19) Europäisches Patentamt
European Patent Office
Office européen des brevets

(11) EP 3 057 690 B1

(12) EUROPÄISCHE PATENTSCHRIFT

(45) Veröffentlichungstag und Bekanntmachung des Hinweises auf die Patenterteilung:
**01.08.2018 Patentblatt 2018/31**

(21) Anmeldenummer: **14777510.0**

(22) Anmeldetag: **11.09.2014**

(51) Int Cl.:
***B01D 71/20*** $^{(2006.01)}$ ***C12N 7/00*** $^{(2006.01)}$

(86) Internationale Anmeldenummer:
**PCT/EP2014/002463**

(87) Internationale Veröffentlichungsnummer:
**WO 2015/055269 (23.04.2015 Gazette 2015/16)**

# (54) SULFATIERTE CELLULOSEHYDRAT-MEMBRAN, VERFAHREN ZU IHRER HERSTELLUNG UND VERWENDUNG DER MEMBRAN ALS ADSORPTIONSMEMBRAN FÜR DIE VIRENAUFREINIGUNG

SULFATED CELLULOSE HYDRATE MEMBRANE, METHOD FOR PRODUCING SAME, AND USE OF THE MEMBRANE AS AN ADSORPTION MEMBRANE FOR A VIRUS PURIFICATION PROCESS

MEMBRANE D'HYDRATE DE CELLULOSE SULFATÉE, PROCÉDÉ DE FABRICATION DE CELLE-CI ET UTILISATION DE LA MEMBRANE COMME MEMBRANE D'ADSORPTION POUR LA PURIFICATION DE VIRUS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.10.2013 DE 102013017014**

(43) Veröffentlichungstag der Anmeldung:
**24.08.2016 Patentblatt 2016/34**

(73) Patentinhaber: **Sartorius Stedim Biotech GmbH
37079 Göttingen (DE)**

(72) Erfinder:
- **VILLAIN, Louis
30449 Hannover (DE)**
- **HÖRL, Hans-Heinrich
37120 Bovenden (DE)**
- **BRUMM, Christian
37249 Neu-Eichenberg (DE)**

(74) Vertreter: **Müller-Boré & Partner
Patentanwälte PartG mbB
Friedenheimer Brücke 21
80639 München (DE)**

(56) Entgegenhaltungen:
**EP-A1- 1 982 727 WO-A1-95/32793
DE-A1-102011 012 569**

- **LARS OPITZ ET AL: "Sulfated membrane adsorbers for economic pseudo-affinity capture of influenza virus particles", BIOTECHNOLOGY AND BIOENGINEERING, Bd. 103, Nr. 6, 15. August 2009 (2009-08-15), Seiten 1144-1154, XP055018567, ISSN: 0006-3592, DOI: 10.1002/bit.22345**
- **MICHAEL W. WOLFF ET AL: "Purification of cell culture-derived modified vaccinia ankara virus by pseudo-affinity membrane adsorbers and hydrophobic interaction chromatography", BIOTECHNOLOGY AND BIOENGINEERING, Bd. 107, Nr. 2, 1. Oktober 2010 (2010-10-01), Seiten 312-320, XP055018569, ISSN: 0006-3592, DOI: 10.1002/bit.22797**
- **LUS RAIADO-PEREIRA ET AL: "Grafting hydrophobic and affinity interaction ligands on membrane adsorbers: A close-up view by X-ray photoelectron spectroscopy", SEPARATION AND PURIFICATION TECHNOLOGY, ELSEVIER SCIENCE, AMSTERDAM, NL, Bd. 93, 22. März 2012 (2012-03-22), Seiten 75-82, XP028502499, ISSN: 1383-5866, DOI: 10.1016/J.SEPPUR.2012.03.028 [gefunden am 2012-03-29]**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 057 690 B1

**Beschreibung**

Die vorliegende Erfindung betrifft eine sulfatierte Cellulosehydrat-Membran, ein Verfahren zu ihrer Herstellung und die Verwendung der Membran als Adsorptionsmembran für die Virenaufreinigung.

Influenza (Virusgrippe) ist eine hoch ansteckende akute Infektion der oberen und unteren Atemwege, die durch Influenzaviren ausgelöst wird. Influenza tritt weltweit insbesondere in den Wintermonaten epidemisch auf, wobei einzelne Viren auch Pandemien auslösen können. Derzeitige Strategien, Influenzaausbrüche zu kontrollieren, basieren auf prophylaktischen Impfungen in Kombination mit antiviralen Behandlungen, z.B. mit Neuraminidase-Inhibitoren. Ein Großteil der saisonalen Humaninfluenza-Impfstoffe sind inaktivierte trivalente Split-/Spalt-Vakzine. Diese setzen sich aus viralen Antigenen, den Hüllglykoproteinen Hämagglutinin (HA) und Neuraminidase (NA) von drei verschiedenen Influenza-Virussubtypen, zusammen. Die jeweiligen Antigene werden aus aufgereinigten Viruspartikeln durch Membransolubilisierung mit Detergenzien (z.B. CTAB (Cetyltrimethylammoniumbromid) oder Tween 20 (Polysorbat 20)) gewonnen.

In den klassischen Produktionsprozessen werden die Influenzaviren in bebrüteten Hühnereiern produziert. Neben der begrenzten Skalierbarkeit dieses Prozesses und der damit verbundenen Probleme, den steigenden Bedarf des weltweiten Marktes zu decken, gibt es systembedingte Schwierigkeiten, einzelne pandemische Influenzaviren in Hühnereiern zu produzieren (z.B. H5N1). Schließlich hat auch die Problematik der Allergieausbildung gegen Hühnereiweiß dazu geführt, dass seit mehreren Jahren Produktionsprozesse mit Säugerzellen in Bioreaktoren etabliert werden. Nach der Kultivierung von Viren in Zellkulturen ist es notwendig, die Viren von den Kontaminanten (z.B. Wirtszellproteinen, DNS) zu trennen, um sie in reiner Form weiterzuverwenden. Weiterhin ist es von Vorteil, infektiöse von nichtinfektiösen Molekülen bzw. Partikeln zu trennen. In biopharmazeutischen Produktionsprozessen treten Desoxyribonukleinsäure (DNS) und andere Proteine der Wirte kultivierungsbedingt als Nebenprodukte auf. Diese zählen im Allgemeinen zu den Kontaminanten und müssen während der Aufarbeitung vom Endprodukt entfernt werden. Auch bei der Produktion von Viren werden DNS-Fragmente der Wirte oder Viren selbst freigesetzt, welche entfernt werden müssen.

Die Adsorption von Viren an festen Phasen durch chromatographische Aufreinigung hat eine große Bedeutung in der Virusaufreinigung, vor allem im Prozessmaßstab. Diese ist dadurch gekennzeichnet, dass Moleküle am Adsorbermaterial (Ionenaustauscher, hydrophobe oder hydrophile Adsorbenzien) gebunden werden und dann in einem späteren Schritt in einer aufgereinigten Form eluiert werden können. Hierbei ist es wichtig, dass der Adsorptionsvorgang reversibel verläuft, damit hohe Ausbeuten erreicht werden. Es kann entweder eine bloße Anreicherung oder eine Auftrennung in mehrere Zielsubstanzen durchgeführt werden, wobei im letzteren Fall entweder die Adsorption, die Desorption oder beide selektiv erfolgen können. Der Hauptnachteil der dafür eingesetzten chromatographischen Medien liegt in deren relativ niedriger Selektivität, und die in Lösung vorhandenen Kontaminanten (DNS-Fragmente und Wirtszellproteine) können auch am Adsorbermaterial binden, sodass lange Prozessentwicklungszeiten erforderlich sind, um die optimalen Bindungs- und Elutionsbedingungen (pH-Wert, Ionenstärke und Auswahl der Puffersysteme) zu ermitteln.

Eine erhöhte Selektivität bietet die Affinitätschromatographie. Aus dem Stand der Technik ist bekannt, dass sulfatierte Cellulosematrizen eine erhöhte Selektivität gegenüber Influenzaviren und Heparin bindenden Proteinen aufweisen.

EP 0 053 473 A1 offenbart Cellulosesulfat-Salze mit heparinähnlicher Wirkung zur Verwendung als Antikoagulanzien. Für eine ausreichend hohe antikoagulierende Wirkung, verbunden mit erwünschter hoher Langzeitstabilität unter physiologischen Bedingungen, liegt der Sulfatierungsgrad (der Grad der Substitution der OH-Gruppen durch Sulfatgruppen am C2-, C3- und C6-Atom der Glycopyranoseeinheiten) der Cellulose zwischen 0,8 und 2,6. Die Cellulosesulfat-Salze sind nach drei verschiedenen Methoden erhältlich: durch Umsetzung mit Chlorschwefelsäure in Anwesenheit eines Amins, durch Umsetzung mit $SO_3$-Amid-Komplexen, wobei als Amid beispielsweise Dimethylformamid eingesetzt werden kann, oder durch Umsetzung mit $SO_3$-Amin-Komplexen, wobei als Amin beispielsweise Pyridin verwendet werden kann. Bei letztgenannter Variante wird die Cellulose mit einem $SO_3$-Pyridin-Komplex bei Raumtemperatur für 30 bis 35 min umgesetzt und nachfolgend mit NaOH neutralisiert.

EP 1 698 641 A1 offenbart Cellulosesulfat-Salze als therapeutische Wirkstoffe gegen Hauterkrankungen, wie atopische Ekzeme. Die Cellulosesulfat-Salze zeichnen sich durch eine Hemmwirkung auf die Hyaluronidase aus und haben einen Schwefelgehalt von 6,5 bis 19,0 Gew.-% bezogen auf das Gesamtgewicht des Cellulosesulfat-Salzes. Bei der Herstellung wird kristalline Cellulose zunächst in einem Solvens, wie Pyridin, Dimethylsulfoxid oder Dimethylformamid, vorgequollen. Die resultierende Mischung wird einem Sulfatierungsreagenz, ausgewählt aus der Gruppe Chlorsulfonsäure, Piperidin-Schwefelsäure-Komplex, $SO_3$-Pyridin-Komplex, $SO_3$-Trimethylamin-Komplex oder Schwefelsäureanhydrid-Dimethylformamid-Komplex, zugesetzt, wobei der letztgenannte Komplex bevorzugt ist.

Die beiden vorgenannten Dokumente EP 0 053 473 A1 und EP 1 698 641 A1 offenbaren keine chromatographischen Trennmaterialien für die Aufreinigung von Viren. Solche Trennmaterialien sind auf Gel-Basis kommerziell erhältlich und werden für die Aufreinigung von den oben genannten Molekülen und Viren verwendet. Die Anwendung dieser Medien zur Reinigung von Viren liefert oft niedrige Bindungskapazitäten für die Viren und niedrige Virusausbeuten. Viren können eine Größe bis zu 500 nm aufweisen, und partikuläre Chromatographie-Gele mit Porengrößen im Bereich von 30 bis 400 nm sind daher eher ungeeignet. Üblicherweise können Viren dann nur auf der äußeren Oberfläche der Partikel binden, was die geringen Bindungskapazitäten erklärt.

Die Sulfatierung chromatographischer Gele aus Cellulose, Dextran oder Agarose und die Verwendung dieser sulfatierten Gele für die Aufreinigung von Influenzaviren sind aus dem Stand der Technik bekannt. Beispielsweise offenbart RD 298 025 A Affinitätschromatographie-Gele, welche durch Sulfatierung von "Cellulofine GH-25", "Sepharose CL-6B" bzw. "Sephadex G-50" mit einem Chlorsulfonsäure-Pyridin-Komplex bei 65 bis 70 °C, nachfolgende Neutralisation mit NaOH und Waschen mit phosphatgepufferter Kochsalzlösung herstellbar sind. Die Gele sind zur Reinigung von Proteinen, wie Hepatitis-B-Antigenen, HIV-1- und HIV-3-Viren, SV40-T-Antigenen, Blutgerinnungsfaktoren 7, 8, 9 und 11, Nukleinsäure-Polymerasen, Interferonen oder Lysozym geeignet.

EP 0 171 086 A2 beschreibt ein Verfahren zur Sulfatierung von (teil)kristallinen Polysaccharidgelen auf Basis von Agarose, Dextran oder Cellulose mit Chlorsulfonsäure oder wasserfreier Schwefelsäure in Pyridin bei 65 bis 70 °C. Der Sulfatierungsgrad der Polysaccharidgele im Allgemeinen liegt zwischen 0,1 und 40 %, während der Sulfatierungsgrad der Cellulose-Gele im Speziellen zwischen 0,1 und 5 % liegt. Mit den sulfatierten Cellulose-Gelen können Influenzaviren bzw. -antigene aus Hühnerembryo-Zellkulturen aufgereinigt werden.

WO 2008/039136 A1 offenbart eine poröse Polysaccharidmatrix, bevorzugt aus Agarose, an welche "Extender"-Moleküle aus 500-kDa-Dextran gebunden sind, wobei an die Dextran-Moleküle ihrerseits Sulfatgruppen als Liganden für die Aufreinigung von Viren gebunden sind. Die Matrix wird für die Abtrennung von Viren, bevorzugt Influenzaviren, von DNS-Kontaminanten verwendet, wobei die Viren zunächst an der Matrix adsorbiert und nachfolgend mit einem geeigneten Puffer eluiert werden. Zur Herstellung der vorgenannten Matrizen werden zunächst SulfatLiganden an die "Extender"-Moleküle gebunden, bevor die sulfatierten "Extender"-Moleküle auf der Polysaccharidmatrix fixiert werden.

EP 0 171 771 A2 offenbart die Verwendung der aus EP 0 171 086 A2 bekannten sulfatierten Cellulosegele zur Reinigung von Tollwutviren aus Hühnerembryo-Zellkulturen.

EP 0 173 268 A2 offenbart die Verwendung der aus EP 0 171 086 A2 bekannten sulfatierten Cellulosegele zur Reinigung von Glykoproteinen gA und gB als Bestandteile von Herpes-Simplex-Viren der Typen HSV-1 und HSV-2, die aus Lysaten von Säugerzellkulturen gewonnen wurden. Die Reinigung der Glykoproteine gA und gB erfolgt in Anwesenheit eines anionischen bzw. nichtionischen Tensids.

EP 0 171 765 A2 offenbart ein Verfahren zur Reinigung des japanischen Enzephalitis-Virus zur Vakzinherstellung, wobei die aus EP 0 171 086 A2 bekannten sulfatierten Cellulosegele verwendet werden.

Alle vorgenannten Dokumente offenbaren chromatographische Trennmedien, bestehend aus partikulären porösen Gelpartikeln. Hierbei werden Polysaccharidgele als Basismaterial für die Sulfatierungsreaktion verwendet, welche eine Ausschlußgrenze ("Molecular Weight Cut Off", MWCO) von weniger als 107 Da haben. Viren, wie beispielsweise Influenzaviren mit einem Durchmesser größer als 100 nm und einer Molekülmasse (MW) größer als $10^8$ Da, wie von R. W. H. Ruigrok et al. in J. Gen. Virol. (1984), 65, 799-802, beschrieben, können nur begrenzt in die Poren der beschriebenen porösen Trennmaterialien eindringen, wodurch nur ein Teil der Bindungskapazität dieser Trennmaterialien ausgenutzt werden kann.

Adsorptionsmembranen haben im Unterschied zu partikulären Adsorbentien wesentlich größere Poren, die für Viren vollkommen zugänglich sind. Außerdem bieten sie die Möglichkeit, durch Anlegung einer hydraulischen Druckdifferenz zwischen ihren beiden Hauptoberflächen eine Durchströmung mit dem Medium zu erzwingen, wodurch anstelle eines rein diffusiven Transportes der Adsorbenden in Richtung eines Konzentrationsgradienten ins Innere des Adsorbens ein konvektiver Transport erreicht wird, der bei hohem Durchfluss sehr viel rascher erfolgen kann. Dadurch kann ein weiterer, den partikulären Adsorbentien inhärenter Nachteil, der als "Diffusionslimitierung" bezeichnet wird, vermieden werden, der darin besteht, dass mit zunehmender Partikelgröße des Adsorbens und zunehmender Molmasse des Adsorbenden die erforderliche Zeit zur Einstellung des Adsorptionsgleichgewichts erheblich zunimmt, was sich in einer Verschlechterung der Kinetik auswirkt. Aus diesem Grund werden bei der Aufreinigung von beispielsweise Influenzaviren mit chromatographischen Gelen typischerweise nur geringe Flussraten erreicht.

Sulfatierte, auf Cellulose basierende Membranadsorber wurden schon durch Sulfatierung einer Cellulosehydrat-Membran erfolgreich hergestellt, wobei die Sulfatierung typischerweise durch Reaktion der Cellulose-Membran mit einem Lewisbase-$SO_3$-Komplex in einem für die Reaktion geeigneten Lösungsmittel erfolgt. Im Vergleich mit herkömmlichen sulfatierten Polysaccharidgelen weisen sulfatierte Membranadsorber folgende Vorteile auf:

- Konvektiver Fluss im Vergleich zu diffusivem "Fluss" bei partikulären Trennmaterialien
- Höherer Fluss möglich
- Bessere Zugänglichkeit der Liganden und höhere Bindungskapazität für Viren
- Einfachere Skalierbarkeit, d.h. Maßstabsvergrößerung von kleinvolumigen Membranadsorbern zu großvolumigen Membranadsorbern.

Da die Kapazität von der Ligandendichte und von der verfügbaren Bindungsfläche abhängt, wird diese durch die Erniedrigung der Membranporengröße typischerweise erhöht, was sich aber in Bezug auf Fluss und Verblockungsverhalten negativ auswirken kann.

US 5,667,684 offenbart mikroporöse Membranen zur Entfernung von HIV-Viren aus Blutplasma, die aus einem hydrophoben Basismaterial, z.B. Polypropylen, Polyethylen oder Polyvinylidenfluorid bestehen, auf welches eine Kette aus Alkoxyalkylacrylaten, Glycidylacrylaten oder Acrylamiden aufgepfropft ist. Auf dieser intermediären Pfropfschicht wird durch kovalente Bindung sulfatierte Cellulose immobilisiert, wobei z.B. freie OH-Gruppen der sulfatierten Cellulose mit funktionellen Gruppen, z.B. Epoxidgruppen, der Wiederholungseinheiten der Pfropfkette umgesetzt werden. Die Entfernung der HIV-Viren erfolgt überwiegend durch die sulfatierten Celluloseeinheiten.

US 8,173,021 B2 offenbart ein Herstellungsverfahren für sulfatierte, nicht vernetzte Cellulosemembranen mit einem Sulfatierungsgrad (Sulfonylgruppengehalt) zwischen 0,5 und 15 %, bei welchem Cellulosemembranen bei höchstens 40 °C mit einem Chlorsulfonsäure-Pyridin-Komplex umgesetzt werden. Der Chlorsulfonsäure-Pyridin-Komplex wird durch Zugabe von Chlorsulfonsäure zu Pyridin bei maximal 0 °C, nachfolgende Umsetzung bei 60 °C und Abkühlung auf maximal 40 °C hergestellt. Die aus nicht vernetzten, regenerierten Cellulosemembranen (z.B. RC-55-Membranen der Fa. Whatman) herstellbaren sulfatierten Cellulosemembranen werden in der Affinitätschromatographie zur Reinigung von proteinhaltigen Virusbruchstücken oder zur Reinigung intakter Viruspartikel verwendet, die nachfolgend in der Influenza-Vakzinherstellung eingesetzt werden.

L. Opitz et al., Biotechnology and Bioengineering, 103 (6), 2009, 1144-1154 offenbaren die Herstellung sulfatierter Cellulosemembranen durch Umsetzung von verstärkten, nicht vernetzten Cellulosemembranen mit einer Lösung von Chlorsulfonsäure in Pyridin bei 37 °C für 12 Stunden. Der Schwefelgehalt der sulfatierten Cellulosemembranen liegt mit 16 $\mu$g Schwefel / g getrockneter Membran signifikant niedriger als bei dem partikulären Adsorbens Cellufine® Sulfate ($\geq$ 700 $\mu$g Schwefel / g getrockneter Membran). Die sulfatierten Cellulosemembranen werden zur Aufreinigung von drei Influenzavirusstämmen, d.h. H1N1-, H3N2- und "B/Malaysia/2506/2004"-Viren, verwendet, wobei unerwünschte Kontaminanten, wie doppelsträngige DNS oder Zellbestandteile, aus der für die Virenproduktion verwendeten Zellkulturlösung effektiv abgetrennt werden. Mit steigender NaCl-Konzentration in der für die Reinigung verwendeten Pufferlösung (150nM versus 50mM) sinkt das Adsorptionsvermögen der Membranen für die Viren. Die sulfatierten Cellulosemembranen erlauben im Vergleich zu KationenaustauscherMembranen, wie Sartobind® S75 oder Sartobind® C75, eine verbesserte Abreicherung von doppelsträngiger DNS und erlauben eine höhere Virus-Ausbeute als Chromatographiesäulen auf Basis von Cellufine® Sulfate.

US 2012/0171750 A1 untersucht den Einfluss der Reaktionstemperatur bei der Herstellung der aus dem vorstehend genannten Artikel von L. Opitz et al. bekannten sulfatierten Cellulosematrizen auf den Sulfatierungsgrad der Cellulosematrix, das Adsorptionsvermögen für MVA-Viren und die Kontamination der gereinigten MVA-Viruszubereitung mit DNS aus der Zellkulturlösung. Mit steigender Reaktionstemperatur bei der Sulfatierung (35, 40 bzw. 45 °C) steigt der Sulfatierungsgrad der Cellulosematrix von 5,3 auf 13 Gew.-%, bezogen auf das Gewicht des Cellulose-Polymerrückgrats, während der DNS-Anteil in der gereinigten Viruszubereitung von 7,4 auf 17 % steigt und die Ausbeute an gereinigten MVA-Viren von 66 auf 80 % steigt. Die Reinigung der Viruszubereitung mittels sulfatierter Cellulosematrizen kann mit einem zusätzlichen Schritt einer hydrophoben Interaktionschromatographie kombiniert werden, bei welcher Chromatographiematrizen mit Phenyl-, Butyl- oder Hexylliganden eingesetzt werden.

Mit dem aus US 8,173,021 B2 bekannten Verfahren kann trotz der Verwendung von relativ engen Membranen (RC-55-Membranen der Firma Whatman mit einer Porengröße von 0,45 $\mu$m) kein Sulfatierungsgrad von größer als 15 % erreicht werden. Die gewählte Porengröße ist außerdem für die Bindung von Viren mit einem Durchmesser von mehr als 100 nm ungünstig, da ein Flussverlust und eine Verblockung der Membran während der Adsorption der Viren stattfinden kann.

Der vorliegenden Erfindung liegt daher die Aufgabe zugrunde, Membranen für die Virenaufreinigung, wie beispielsweise die Influenzavirus-Aufreinigung, bereitzustellen, die einerseits eine hohe Selektivität bezüglich der zu isolierenden Zielverbindungen aufweisen, und andererseits eine effektive Abtrennung unerwünschter Kontaminanten, wie doppelsträngige DNS oder Zellbestandteile aus der für die Virenproduktion verwendeten Zellkulturlösung, bei hohen Flussraten ermöglichen.

Diese Aufgabe wird durch die in den Ansprüchen gekennzeichneten Gegenstände gelöst.

Insbesondere stellt die vorliegende Erfindung eine sulfatierte Cellulosehydrat-Membran bereit, umfassend eine vernetzte Cellulosehydrat-Matrix mit Poren, die sich von einer Hauptoberfläche zur anderen Hauptoberfläche der Membran erstrecken, wobei die Cellulosehydrat-Membran auf ihren inneren und äußeren Oberflächen Sulfatliganden zur adsorptiven Stofftrennung aufweist.

Gemäß der vorliegenden Erfindung handelt es sich bei der sulfatierten Cellulosehydrat-Membran um eine Adsorptionsmembran. Als Adsorptionsmembranen werden flächige Adsorbentien mit von der einen Seite zur anderen Seite durchgehenden Poren bezeichnet. Adsorbentien sind poröse Feststoffe, die über als Liganden bezeichnete funktionelle Oberflächengruppen mit bestimmten Komponenten von Fluiden selektiv Bindungen eingehen können. Zielsubstanz(en) und/oder Kontaminant(en) werden erfindungsgemäß als Adsorbenden bezeichnet, wobei es sich auch um mehrere unterschiedliche Substanzen handeln kann. Adsorbenden können Einzelmoleküle, Assoziate oder Partikel sein, bei denen es sich im Falle der Zielsubstanz(en) vorzugsweise um Viren handelt.

Gemäß der vorliegenden Erfindung erfolgt die Vernetzung der Cellulosehydrat-Matrix, bevor diese in einem anschließenden Prozeßschritt sulfatiert wird. Überraschenderweise wurde festgestellt, dass die vorherige Vernetzung der zu sulfatierenden Cellulosehydrat-Membran zu einer erhöhten Permeabilität für Reverseosmose-Wasser (RO-Wasser), zu einer erhöhten Sulfatligandendichte, zu einer erhöhten Bindungskapazität und zu einer erhöhten Bindungsselektivität für die zu isolierenden Zielverbindungen, insbesondere für Influenzaviren, führt, wobei diese vorteilhaften Effekte nicht durch Verwendung von sulfatierten Cellulosemembranen erreicht werden können, die vor ihrer Sulfatierung nicht vernetzt werden. Im Gegensatz zu US 8,173,021 B2 und US 2012/0171750 A1 können durch die Sulfatierung einer vernetzten Cellulose-Membran überraschenderweise deutlich höhere Sulfatligandendichten erreicht werden, als sie nach dem Stand der Technik erzielbar sind.

Erfindungsgemäß wird die Sulfatligandendichte durch den Sulfatierungsgrad bestimmt, wobei der Sulfatierungsgrad der Cellulosehydrat-Matrix 15 Gew.-% oder mehr, und besonderes bevorzugt mehr als 20 Gew.-% beträgt, was zu einer verbesserten Selektivität zugunsten der Virusgewinnung führt. Erfindungsgemäß wird unter Sulfatierungsgrad der Massenanteil an Sulfatgruppen an der sulfatierten Cellulosehydrat-Matrix verstanden. Die quantitative Bestimmung des Sulfatierungsgrades erfolgt durch Titration, wobei die Stoffmenge an Sulfatgruppen auf der Membran beispielsweise mit Natronlauge ermittelt werden kann.

Gemäß der vorliegenden Erfindung wird der Sulfatierungsgrad mit folgender Formel bestimmt:

$$\text{Sulfatierungsgrad in Gew.-\%} = (n_{Sulfat} \times M(SO_3) / (m_{Cellulose} + (n_{Sulfat} \times M(SO_3))) \times 100\ \%$$

mit

$m_{Cellulose}$ = Masse in $\mu$g an Cellulose pro $cm^2$ der Membran

und der Sulfatgruppendichte $n_{Sulfat}$, die anhand folgender Formel bestimmt wird:

$$\text{Sulfatgruppendichte: } n_{Sulfat}\ (\mu mol/cm^2) = c(NaOH) \times t(NaOH) \times V(NaOH) \times 1000/A$$

mit

| | |
|---|---|
| c(NaOH) | Stoffmengenkonzentration des Titriermittels Natronlauge in mol/l |
| V(NaOH) | Volumen in ml des verbrauchten Titriermittels am Äquivalenzpunkt (pH 7) |
| t(NaOH) | Korrekturfaktor des Titriermittels |
| 1000 | Umrechnungsfaktor von mol/l auf $\mu$mol/ml |
| A | aktive Filterfläche in $cm^2$ |
| und mit $M(SO_3)$ | Molare Masse von $SO_3$ in $\mu g/\mu mol$. |

Im Vergleich zur Sulfatierung von unvernetzten Cellulose-Membranen kann gemäß der vorliegenden Erfindung durch die Vernetzung der Cellulose-Membranen die Sulfatligandendichte überraschenderweise um einen Faktor von bis zu 30 im Vergleich zu den Vergleichsbeispielen aus dem Stand der Technik (Proben 1 und 3 der Tabelle 2) erhöht werden. Dies ist sogar mit Cellulosemembranen möglich, die eine mittlere Porengröße von mehr als 0,45 $\mu$m aufweisen.

Eine zusätzliche überraschende Konsequenz der vorherigen Vernetzung der Cellulosehydrat-Membranen ist die damit verbundene Permeabilitätserhöhung und Abnahme der Bindungskapazität für kleine Proteine oder Kontaminanten, wie Wirtszellproteine, trotz steigender Sulfatligandendichte, was in einer verbesserten Selektivität zugunsten der Virusgewinnung resultiert.

Wie im Weiteren beschrieben wird, kann das Verfahren zur Herstellung der erfindungsgemäßen Membran in zwei Schritten durchgeführt werden: Vernetzung der Membran und Sulfatierung der Membran.

Durch die Art des Vernetzungsmittels, die Konzentration des Vernetzungsmittels, die Konzentration eines gegebenenfalls eingesetzten Vernetzungskatalysators, die Vernetzungsdauer, gegebenenfalls die Art und die Konzentration eines inerten organischen Lösungsmittels und/oder die Vernetzungstemperatur kann der Vernetzungsgrad, die Porengröße und als Konsequenz die Permeabilität der Membran und die Zugänglichkeit der Hydroxylgruppen für die weitere Sulfatierungsreaktion gesteuert werden.

Gemäß einer bevorzugten Ausführungsform der erfindungsgemäßen, sulfatierten Cellulosehydrat-Membran beträgt der Vernetzungsgrad zwischen 0,05 und 0,5, wobei als Maß für den Vernetzungsgrad (VG) der mittlere Substitutionsgrad der Anhydroglucose-Einheiten der Cellulose durch Reaktion mit dem difunktionellen Vernetzer gewählt wird. Dabei wird der Vernetzungsgrad erfindungsgemäß, wie in WO 95/32793 A1 beschrieben, definiert als:

$$VG = 3{,}24 \times \Delta M/M_V$$

mit

VG: Vernetzungsgrad der erfindungsgemäßen Membran,

$\Delta$M: Massenzuwachs der erfindungsgemäßen Membran durch die Vernetzung in % der Ausgangsmasse der Ausgangsmembran,

$M_V$: Molmasse des Vernetzers.

Geht man von einem difunktionellen Vernetzer aus und unterstellt, dass dieser je zwei Cellulose-Monomereinheiten verbindet, dann können maximal drei Vernetzermoleküle mit zwei Cellulose-Monomereinheiten reagieren. Diese haben zusammen ein Molgewicht von 324 g/mol. Der theoretische maximal mögliche Wert von VG liegt aufgrund der drei vernetzbaren Hydroxylgruppen der Cellulose-Monomereinheit bei maximal 3.

Gemäß der vorliegenden Erfindung eignet sich die sulfatierte Cellulosehydrat-Membran zur adsorptiven Stofftrennung, insbesondere zur effizienten Aufreinigung von Viren. Dies ist insbesondere möglich, wenn die Ausschlußgrenze der erfindungsgemäßen sulfatierten Cellulosehydrat-Membran so gewählt wird, dass die Zielsubstanzen nicht die Membranoberfläche blockieren, wodurch eine hohe Flussrate aufrecht erhalten werden kann. In Abhängigkeit von der gewünschten Zielsubstanz kann die Ausschlußgrenze derart eingestellt werden, dass die Zielsubstanz im Inneren der sulfatierten Cellulosehydrat-Membran adsorbiert wird und anschließend aus dieser eluiert werden kann. Vorzugsweise beträgt die mittlere Porengröße der erfindungsgemäßen Membranen zwischen 0,5 und 5,0 $\mu$m und besonders bevorzugt zwischen 1,0 und 3,0 $\mu$m.

**Ausgangsmembran:**

Die in dem erfindungsgemäßen Verfahren als Ausgangsmembran verwendete Cellulosehydrat-Membran mit einer mittleren Porengröße von 0,1 bis 20 $\mu$m, vorzugsweise 0,5 bis 15 $\mu$m und mehr bevorzugt von 1 bis 10 $\mu$m wird durch ein übliches, auf dem Fachgebiet bekanntes Herstellungsverfahren hergestellt, wie beispielsweise in L. J. Zeman et al., "Microfiltration and ultrafiltration principles and applications", Marcel Dekker 1996, "Part I", "Chapter 3.1", DE 103 26 741 A1 und DE 34 47 625 A1 beschrieben. Zur Bestimmung der mittleren Porengröße wurde ein "Capillary Flow Porometry Test" durchgeführt. Details sind der Bedienungsanleitung für das "Capillary Flow Porometer 6.0", CAPWIN Software System, Fa. Porous Materials Inc., zu entnehmen.

Gemäß der vorliegenden Erfindung ist es bevorzugt, dass Celluloseester-Membranen, welche gegebenenfalls einer nachstehend beschriebenen Vorbehandlung unterzogen werden, mit einem geeigneten Verseifungsmedium verseift werden, wodurch sich die Cellulosehydrat-Membran ausbildet. Je nach der Art des Vorbehandlungsmediums kann die Celluloseester-Membran trocken oder naß im Verseifungsschritt eingesetzt werden.

Celluloseester-Membranen können aus Cellulosemonoacetat, Cellulosediacetat, Cellulosetriacetat, Cellulosepropionat, Cellulosebutyrat und Celluloseacetobutyrat oder anderen geeigneten Celluloseestern oder Cellulosenitrat, Methylcellulose oder Ethylcellulose sowie Gemischen davon, aufgebaut sein, wobei Celluloseacetate, insbesondere Cellulosediacetat, bevorzugt sind bzw. ist. Dabei kann die Celluloseester-Membran in einem geeigneten Medium vor dem Verseifungsschritt vorbehandelt werden, wobei das Vorbehandlungsmedium ein oder mehrere Additiv(e) enthält, das bzw. die eine gegenüber einem Celluloseester lösende oder weichmachende Wirkung aufweist bzw. aufweisen. Geeignete Additive sind vor allem Säuren, insbesondere Carbonsäuren, wie Essigsäure, und wasserlösliche Weichmacher für Celluloseester, wie Diacetin, Triacetin und Sulfolan.

Das Verseifungsmedium weist vorzugsweise eine alkalische Verbindung, bevorzugt ein Alkalimetallhydroxid auf. Besonders bevorzugt ist es, eine wässrige Lösung aus Natrium-, Kalium- oder Lithiumhydroxid zu verwenden. Es können auch Gemische aus einem Alkalimetallhydroxid und anderen alkalischen Verbindungen wie Alkalimetallcarbonat, wie Natriumcarbonat, Kaliumcarbonat, Cäsiumcarbonat, Natriumhydrogencarbonat, und/oder Natriumtriphosphat, Kaliumtriphosphat, Natriumsilikat und Kaliumsilicat eingesetzt werden.

Die erhaltene Cellulosehydrat-Membran kann jede geeignete Dicke aufweisen. Vorzugsweise liegt die Membrandicke im Bereich zwischen 50 und 500 $\mu$m, noch bevorzugter im Bereich zwischen 100 und 300 $\mu$m. Die erhaltene Cellulosehydrat-Membran kann als Flachmembran oder zylindrisch ausgebildet sein. Zylindrische Membranen werden als Membranhohlfasern, Membrankapillaren oder Membranschläuche bezeichnet.

**Vernetzung:**

Das Vernetzungsmittel weist mindestens zwei funktionelle Gruppen im Molekül auf, die mit den Hydroxylgruppen der Cellulose-Membran reagieren und somit eine Vernetzung der Cellulose-Membran ermöglichen. Beispielsweise erfolgt die Vernetzung der Cellulose-Membran über die (intermolekulare) Verbindung von zwei Cellulosepolymerketten oder über die (intramolekulare) Verbindung von Wiederholungseinheiten einer Cellulosepolymerkette durch das vorgenannte Vernetzungsmittel mit mindestens zwei funktionellen Gruppen, welche gegenüber HydroxylGruppen der Cellulosepolymerketten reaktiv sind. Die verwendbaren Vernetzungsmittel unterliegen grundsätzlich keinen besonderen Beschränkungen und ein Fachmann ist in der Lage, sie im Hinblick auf die Reaktionsbedingungen der nachfolgenden Sulfatierung auszuwählen. Es ist jedoch bevorzugt, in dem Vernetzungsschritt eine Diepoxidverbindung oder auch andere, mit Hydroxylgruppen von Cellulose reaktive Verbindungen mit mindestens zwei reaktiven funktionellen Gruppen, wie Diisocyanat, Epichlorhydrin, Epibromhydrin, Dimethylharnstoff, Dimethylethylenharnstoff, Dimethylchlorsilan, Bis-(2-hydroxyethylsulfon), Divinylsulfon, Alkylendihalogenid, Hydroxyalkylendihalogenid und Diglycidylether zu verwenden. Besonders bevorzugt wird in dem Vernetzungsschritt eine Diepoxidverbindung verwendet.

Aus der Gruppe der Diglycidylether sind 1,4-Butandioldiglycidylether, Ethylenglycoldiglycidylether, Glycerindiglycidylether und Polyethylenglycoldiglycidylether bevorzugt.

Besonders bevorzugt ist die Verwendung von 1,4-Butandioldiglycidylether oder Ethylenglycoldiglycidylether als Vernetzungsmittel.

Optional kann ein Gemisch von unterschiedlichen Vernetzungsmitteln verwendet werden.

Die Vernetzung kann in einem wässrigen Medium, in einem organischen Lösungsmittel oder auch in einem Gemisch aus Wasser und einem organischen Lösungsmittel stattfinden. Vorzugsweise wird die Vernetzung in einem wässrigen Medium durchgeführt.

Ferner ist es bevorzugt, einen Vernetzungskatalysator, wie Natrium- oder Kaliumhydroxid, zur Beschleunigung der Vernetzung von Cellulose mit dem Vernetzungsmittel zu verwenden.

Die Temperatur des verwendeten Mediums in dem Vernetzungsschritt kann von etwa 4 °C bis zum Siedepunkt des Vernetzungsmediums betragen, wobei eine Temperatur in einem Bereich von 5 °C bis etwa 85 °C bevorzugt ist. Besonders bevorzugt ist eine Temperatur von 20 °C bis 40 °C.

Üblicherweise beträgt die Vernetzungsdauer wenige Minuten bis mehrere Tage, wobei eine Vernetzungsdauer von 12 Stunden bis 7 Tagen bei 15 bis 30 °C bevorzugt ist. Besonders bevorzugt ist eine Vernetzungsdauer von 1 bis 3 Tagen. Bei einer bevorzugten Ausführungsform des erfindungsgemäßen Verfahrens zur Herstellung der erfindungsgemäßen Membran beträgt die Konzentration des Vernetzungsmittels in der Vernetzungslösung zwischen 10 und 30 Gew.-%.

Wie vorstehend beschrieben, beträgt der Vernetzungsgrad der erfindungsgemäßen, sulfatierten Cellulosehydrat-Membran mindestens 0,05, mehr bevorzugt mindestens 0,07 und besonders bevorzugt mindestens 0,1. Die Obergrenze des Vernetzungsgrades liegt vorzugsweise bei 0,5, mehr bevorzugt bei 0,4 und besonders bevorzugt bei 0,3.

Als weiteren Vorteil weisen die erfindungsgemäßen Membranen durch die Vernetzung eine höhere mechanische Stabilität auf und können dadurch höhere Reaktionstemperaturen (bis zu 90 °C) während des nachfolgenden Sulfatierungsprozesses tolerieren.

**Sulfatierung:**

Die Sulfatierung erfolgt durch Reaktion der vernetzten Cellulosehydrat-Membran mit einem Lewisbase-$SO_3$-Komplex. Die Umsetzung kann mit Chlorschwefelsäure in Anwesenheit eines Amins oder durch Umsetzung mit $SO_3$-Amid-Komplexen, wobei als Amid vorzugsweise Dimethylformamid eingesetzt werden kann, oder durch Umsetzung mit $SO_3$-Amin-Komplexen, wobei als Amin vorzugsweise Pyridin oder Trimethylamin verwendet werden kann, erfolgen. Besonders bevorzugt für die Reaktion ist der $SO_3$-Pyridin-Komplex.

Üblicherweise beträgt die Konzentration des $SO_3$-Pyridin-Komplexes in der Sulfatierungslösung 1 bis 40 Gew.-%, besonders bevorzugt sind Konzentrationen zwischen 10 und 30 Gew.-%.

Als Lösungsmittel für die Reaktion mit dem $SO_3$-Amin-Komplex können typische aprotisch polare Lösungsmittel, wie beispielsweise Dimethylformamid (DMF), Dimethylsulfoxid (DMSO), Dimethylacetamid (DMAc), N-Methyl-2-Pyrrolidon oder 2-Pyrrolidon verwendet werden. Besonders bevorzugt ist 2-Pyrrolidon.

Die Reaktionsdauer beträgt zwischen 30 min bis 24 Stunden, besonders bevorzugt sind Reaktionsdauern zwischen 1 und 4 Stunden.

Die Reaktionstemperatur beträgt zwischen 20 bis 90 °C, besonders bevorzugt sind Reaktionstemperaturen von 70 bis 90 °C.

Die vorliegende Erfindung wird anhand der nachstehenden Ausführungsbeispiele und der Figuren 1 bis 3 näher erläutert. Dabei zeigt

- Figur 1 den Einfluss des Vernetzungsgrades auf den Sulfatierungsgrad der erfindungsgemäßen Membran, auf die Permeabilität für RO-Wasser und auf die Bindungskapazität für Lysozym,
- Figur 2 die dynamische Bindungskapazität für Influenzaviren und Lysozym, und
- Figur 3 Durchbruchskurven von mit HCP-Lösung ("Host-cell-protein"-Lösung, Wirtszellprotein-Lösung) beladenen sulfatierten Polysaccharidgelen aus dem Stand der Technik sowie einer erfindungsgemäß sulfatierten Cellulose-Membran.

**Ausführungsbeispiele**

Fünf verschiedene Membranen wurden funktionalisiert, um den Einfluss der Vernetzung zu veranschaulichen. Zwei verschiedene Sulfatierungsmethoden wurden sowohl auf vernetzte als auch auf unvernetzte Cellulosehydrat-Membranen (Vergleichsbeispiele) mit der gleichen Porengröße (Proben 1 bis 4) angewendet. Eine weitere Probe wurde hergestellt, welche nach Beispiel 1 vernetzt und nach Beispiel 2 sulfatiert wurde (Probe 5). Diese Probe unterscheidet sich von den Proben 1 bis 4 dadurch, dass eine engere Ausgangsmembran mit einer mittleren Porengröße von 1,2 μm für die Sulfatierung verwendet wurde. Alle Membranproben wurden gemäß Beispiel 5 bis 8 charakterisiert. Die Ergebnisse sind in Tabelle 2 zusammengefasst. Bei der Probe Sartobind® S handelt es sich um eine Cellulosehydrat-Membran mit Sulfonsäureliganden.

Figur 1 zeigt, dass die Erhöhung des Vernetzungsgrades zur Erhöhung des Sulfatierungsgrades, aber auch zur Erhöhung des Durchflusses für RO-Wasser führt. Außerdem weisen die erfindungsgemäß vernetzten sulfatierten Membranen eine höhere Selektivität für Influenzaviren im Vergleich zu Kontaminanten auf, da sie experimentell eine signifikant niedrigere dynamische Bindungskapazität für kleine negativ geladene Proteine, wie Lysozym, zeigen. Der Sulfatierungsgrad ist in der Figur 1 in Gew.-% angegeben. Tabelle 1 enthält die Angaben zur Zusammensetzung der Vernetzungslösungen, die zur Herstellung der Membranen der Figur 1 verwendet wurden.

Figur 2 zeigt, dass die erfindungsgemäßen Membranen der Proben 2, und 5 deutlich bessere Bindungseigenschaften als die Membranen der Proben 1 und 3, die als Vergleichsbeispiele gewählt wurden, aufweisen, da sie höhere dynamische Bindungskapazität für Influenzaviren aufweisen. Die Probe 4 ist ebenfalls eine Vergleichsprobe. Die Membran der Probe 5 hat im Vergleich zu den Proben 1 und 3 eine mindestens um den Faktor 10 höhere dynamische Bindungskapazität für Influenzaviren. Außerdem beträgt bei den Membranen der Proben 2, 4 und 5 die Bindungskapazität für kleine, positiv geladene Kontaminanten-Proteine, wie Lysozym, nur 1/3 derjenigen der unvernetzten Proben 1 und 3 und der "Sartobind® S"-Membran, welche kationenaustauschende Sulfonatgruppen als Liganden aufweist. Die erfindungsgemäße Membran der Probe 5 weist somit von allen untersuchten Proben die höchste Selektivität für die Bindung von Influenzaviren auf.

Die Angabe "E+12" in Figur 2 bedeutet "x $10^{12}$".

Die in Figur 3 dargestellten Durchbruchskurven mit HCP-Lösung ("Host-cell-protein"-Lösung, Wirtszellprotein-Lösung) zeigen, dass die sulfatierte Membran der Probe 4, anders als herkömmliche sulfatierte Polysaccharidgele, praktisch keine Kontaminanten, wie Wirtszellproteine (HCP), bindet. Hierbei wurden als Vergleich Cellufine® Sulfate von der Firma JNC Corporation und Capto® DeVirS der Firma GE Healthcare verwendet. Bei der erfindungsgemäßen Membran der Probe 4 findet ein sofortiger Durchbruch von Wirtszellproteinen (HCP) statt, was beweist, dass diese Membran kein HCP bindet. Bei den als Vergleichsbeispiel betrachteten sulfatierten Gelen wird erst nach 100 ml Säulenvolumen (CV [ml] = "column volume" in ml) das UV-Signal der ursprünglich eingesetzten HCP-Lösung wieder erreicht, was beweist, dass die sulfatierten Gele in erheblichem Umfang diese Kontaminanten binden. Die vorstehend beschriebene Eigenschaft der erfindungsgemäßen Membranen ist bei der Aufreinigung von Viren von großem Vorteil, da Kontaminanten, wie HCP, an dem Membranmaterial nicht binden können und sich dadurch nach der Elution des Virus nicht im Eluat befinden. Aus diesem Grund kann auf weitere Kontaminantenabreicherungsschritte verzichtet werden, und die Gesamtkosten des Aufreinigungsprozesses werden signifikant reduziert. Hier ist auch eindeutig feststellbar, dass die höhere Sulfatligandendichte bei der erfindungsgemäßen Membran auf die Bindung von Kontaminanten keine negative Auswirkung hat und in erwünschter Weise selektiv nur die Bindungskapazität für Influenzaviren verbessert.

Beispiel 1: Vernetzung von Cellulosemikrofiltermembranen

Die Vernetzungsreaktion findet bei 20 bis 25 °C statt. Zu einer vorgelegten Menge (in g) Umkehrosmosewasser wird 10%ige Natronlauge gegeben und gerührt. Danach wird 1,4-Butandioldiglycidylether (Sigma-Aldrich, Art. Nr.: 220892) zugegeben und gerührt, bis eine homogene Lösung entsteht. Der Grad der Vernetzung kann durch die Konzentration an 1,4-Butandiolglycidylether in der Vernetzungslösung gesteuert werden. Die jeweiligen Mengen an Umkehrosmosewasser, Natronlauge und 1,4-Butandioldiglycidylether (BUDGE) in den hier hergestellten Vernetzungslösungen und die resultierenden Vernetzungsgrade der erfindungsgemäßen Membran sind in Tabelle 1 zusammengefasst.

Tabelle 1: Zusammensetzung der Vernetzungslösungen und resultierender Vernetzungsgrad

| 10% NaOH / [g] | ROW / [g] | BUDGE [g] | Vernetzungsgrad |
|---|---|---|---|
| 6,0 | 94,0 | 0,0 | 0 |
| 6,0 | 93,0 | 1,0 | 0,02 |

(fortgesetzt)

| 10% NaOH / [g] | ROW / [g] | BUDGE [g] | Vernetzungsgrad |
|---|---|---|---|
| 6,0 | 92,0 | 2,0 | 0,03 |
| 6,0 | 89,0 | 5,0 | 0,09 |
| 6,0 | 84,0 | 10,0 | 0,12 |
| 6,0 | 79,0 | 15,0 | 0,19 |

Ein DIN-A4-Bogen trockener Cellulose-Mikrofiltermembran der Sartorius Stedim Biotech GmbH (Typ 10142V) wird mit der Vernetzungslösung vollständig benetzt und anschließend 7 Tage luftdicht in einem Polyethylenbeutel gelagert. Nach beendeter Vernetzungsreaktion wird die Mikrofiltermembran 10 min mit fließendem Umkehrosmosewasser gespült und dann im Umlufttrockenschrank bei 80 °C 10 bis 12 min getrocknet.

Zur Bestimmung des Vernetzungsgrades gemäß obiger Definition wird folgendermaßen vorgegangen: Die unvernetzten Cellulosemembranen vom Typ 10142V werden in einer Sartorius-Trockenwaage (Typ MA100) 5 min bei 110 °C getrocknet und ihr Gewicht $m_A$ ermittelt. Dann erfolgt die Benetzung der Membranen mit der Vernetzungslösung. Es ist zusätzlich eine Blindprobe, die ohne Vernetzungsmittel behandelt wird, zu wiegen, um den Gewichtsverlust durch auswaschbare Bestandteile zu ermitteln. Nach einer Vernetzungsdauer von 7 Tagen werden die Membranen 10 min unter fließendem RO-Wasser gespült, dann in einem Umlufttrockenschrank 10 min bei 80 °C vorgetrocknet und schließlich - wie die Ausgangsmembranen - 5 min auf der Trockenwaage getrocknet und $m_v$ bestimmt. Anhand der ohne Vernetzer behandelten Blindprobe wird die Masse der auswaschbaren Bestandteile $m_B$ bestimmt. Die für die definitionsgemäße Auswertung erforderliche prozentuale Massenzunahme ΔM ergibt sich dann folgendermaßen:

$$\Delta M = 100\% * ((m_V - m_A - m_B)/(m_A - m_B)),$$

wobei

- $m_V$ Masse der vernetzten Membran,
- $m_A$ Masse der unvernetzten Membran und
- $m_B$ Masse der auswaschbaren Bestandteile der Blindprobe ist.

Beispiel 2: Sulfatierung mit 2-Pyrrolidon und Pyridin-Schwefeltrioxid-Komplex

Unter Rühren werden zu 8 g 2-Pyrrolidon 2 g Pyridin-Schwefeltrioxid-Komplex (Sigma-Aldrich, Art.-Nr. 84737-500G) gegeben. Das Ansatzgefäß wird dicht verschlossen. Die Mischung wird nun auf 70 °C temperiert, wobei sich der Pyridin-Schwefeltrioxid-Komplex auflöst und eine ockerfarbene Sulfatierungslösung entsteht.

In ein auf 70 °C temperiertes 80-ml-Wägeglas mit Schliffdeckel werden 3,8 g frisch zubereitete 70 °C heiße Sulfatierungslösung auf den Boden des Reaktionsgefäßes pipettiert. Nacheinander werden kreisförmige trockene Cellulosemikrofiltermembranronden (Durchmesser von 70 mm) der unvernetzten Membran (Vergleichsbeispiel, Ausgangsmembran des Beispiels 1 vom Typ 10142V) sowie der nach Beispiel 1 vernetzten Membran in die Sulfatierungslösung eingelegt; sie benetzen dabei spontan. Das Reaktionsgefäß wird dann umgehend dicht verschlossen.

Das Reaktionsgefäß wird nun für 4 Stunden bei 70 °C im Umlufttrockenschrank gelagert. Danach werden die sulfatierten Cellulosemikrofiltermembranen 10 min mit fließendem Umkehrosmosewasser gespült, 5 min in 100 g 1M-NaCl-Lösung geschüttelt und danach 5 min unter fließendem Umkehrosmosewasser gespült. Die Membranen werden anschließend 5 min in 100 g einer Lösung aus 30 Gew.-% Glycerin und 70 Gew.-% Wasser geschüttelt und danach 10 min bei 80 °C getrocknet.

Beispiel 3: Sulfatierung mit Pyridin und Chlorsulfonsäure

Pyridin (Sigma Aldrich, Art.-Nr. 270970-1L) und Chlorsulfonsäure (Sigma Aldrich, Art.-Nr. 571024-100G) werden vor Verwendung auf -18 °C gekühlt. Unter starkem Rühren werden zu 1000 ml Pyridin tropfenweise 60 ml Chlorsulfonsäure innerhalb von 15 min über einen Tropftrichter mit Druckausgleich und Trockenrohr gegeben, wobei eine Temperatur von 5 °C nicht überschritten werden darf; dabei fällt ein weißer Feststoff aus.

Nun wird der Ansatz dicht verschlossen und unter Rühren in einem Wasserbad auf 65 °C erwärmt, bis sich der Feststoff vollständig gelöst hat. Dann wird die so erhaltene Sulfatierungslösung innerhalb einer Stunde auf 40 °C abgekühlt.

Unvernetzte Membranstreifen (Vergleichsbeispiel aus dem Beispiel 1) und Membranstreifen aus dem Beispiel 2 mit einer Breite von 3,3 cm und einer Länge von 21 cm, insgesamt 18 Streifen, werden mit grobem PP-Gewebe aufgewickelt und in eine 500-ml-Polypropylenschraubdeckeldose überführt. Die auf 40 °C temperierte Sulfatierungslösung wird in die Schraubdeckeldose überführt. Die Schraubdeckeldose wird dicht verschlossen.

Das Reaktionsgefäß wird für 20 Stunden bei 40 °C geschüttelt. Anschließend werden die Membranfilterstreifen folgendermaßen gespült und imprägniert:

- 10 min fließendes Umkehrosmosewasser,
- 10 min Schütteln mit 1000 ml 1M NaCl,
- 10 min fließendes Umkehrosmosewasser,
- 10 min in einer Lösung aus 30 Gew.-% Glycerin und 70 Gew.-% Wasser.

Anschließend erfolgt eine Trocknung bei 80 °C für mindestens 12 Stunden.

Beispiel 4: Permeabilitätsbestimmung von Mikrofiltermembranen

Aus sulfatierten Cellulosemikrofiltermembranen gemäß Beispiel 3 werden 47-mm-Ronden ausgestanzt. Diese werden mit Umkehrosmosewasser benetzt und für 5 min unter fließendem Umkehrosmosewasser gespült. Ein Stanzling wird in ein Aufgußfiltrationsgehäuse Sartorius Typ 16249 eingebaut. Die Messung erfolgt bei 20 bis 25 °C bei 0,10 bar Überdruck. Es wird die Zeit gemessen, die 100 g Medium benötigen, um durch den Membranfilter zu strömen. Die Einheit des so ermittelten Durchflusses wird angegeben mit ml/($cm^2$·min·bar). Als Medien werden sowohl Umkehrosmosewasser als auch 10 mM Kaliumphosphatpuffer mit einem pH-Wert von 7 verwendet.

Die Ronden können nach der Bestimmung des Durchflusses zu weiteren Untersuchungen verwendet werden.

Beispiel 5: Quantitative Bestimmung des Sulfatierungsgrades durch Titration

Aus sulfatierten Cellulosemikrofiltermembranen werden mit einem Rundstanzeisen 30-mm-Ronden ausgestanzt. Ein 30-mm-Stanzling wird in ein totvolumenoptimiertes Filtrationsgehäuse eingebaut. Die aktive Filterfläche beträgt 5,7 $cm^2$. Das Filtrationsgehäuse wird luftblasenfrei mit Umkehrosmosewasser gefüllt. Das Filtrationsgehäuse wird an eine Mehrkanalschlauchpumpe der Firma Watson Marlow (Typ 205 U) angeschlossen, deren Zuleitungen luftblasenfrei sind. Die Fördermenge der Schlauchpumpe beträgt circa 5 ml in der Minute. Nacheinander werden folgende Medien zu je 4 min Förderzeit durch den Membranfilter befördert: 1M NaCl, 1M HCl, 1mM HCl, Umkehrosmosewasser.

Unter die Ausgangsseite des Filtrationsgehäuses wird nun ein 40-ml-Becherglas mit Magnetrührstäbchen gestellt. Es wird nun für weitere 4 min 1M NaCl gefördert. Das aufgefangene Eluat wird mittels potentiometrischer Indikation titriert. Das Titriermittel ist 5,0mM Natronlauge. Dazu wird der Verbrauch einer 5mM Natronlauge bis zum Äquivalenzpunkt bei pH 7,0 ermittelt. Die Stoffmenge an verbrauchter Natronlauge ist der Stoffmenge an Sulfatgruppen auf der Membran direkt proportional. Bei gegebener aktiver Fläche der Membran und des Verbrauchs an Natronlauge sowie der Masse an Cellulose pro $cm^2$ lässt sich der Sulfatierungsgrad, welcher der Massenanteil an Sulfatgruppen an der sulfatierten Cellulose ist, berechnen.

Formel:

Sulfatgruppendichte:

$$n_{Sulfat}\,(\mu mol/cm^2) = c(NaOH) \times t(NaOH) \times V(NaOH) \times 1000/A$$

mit

| | |
|---|---|
| c(NaOH) | Stoffmengenkonzentration des Titriermittels Natronlauge in mol/l |
| V(NaOH) | Volumen in ml des verbrauchten Titriermittels am Äquivalenzpunkt (pH 7) |
| t(NaOH) | Korrekturfaktor des Titriermittels |
| 1000 | Umrechnungsfaktor von mol/l auf μmol/ml |
| A | aktive Filterfläche in $cm^2$ |
| und mit $M(SO_3)$ | Molare Masse von $SO_3$ in μg/μmol. |

Formel:

$$\text{Sulfatierungsgrad in Gew.-\%} = (n_{Sulfat} \times M(SO_3) / (m_{Cellulose} + (n_{Sulfat} \times M(SO_3))) \times 100\ \%$$

mit

$m_{Cellulose}$ Masse in µg an Cellulose pro $cm^2$ des Stanzlings.

Die Ergebnisse des Sulfatierungsgrades der Proben 1 bis 5 sind in Tabelle 2 zusammengefasst.

Beispiel 6: Bestimmung der Bindungskapazität für Lysozym:

Membranproben mit einer aktiven Membranfläche von jeweils 17,6 $cm^2$ werden in 35 ml 10mM Kaliumphosphatpuffer (KPi), pH 7,0, dreimal 5 Minuten mit etwa 80 Umdrehungen pro min (Upm) geschüttelt und danach in 35 ml einer Lösung von 2 mg/ml Lysozym in 10mM Kpi, pH 7,6, 12 bis 18 Stunden bei 20 bis 25 °C eingelegt. Anschließend werden die Membranproben 2 x 15 Minuten in jeweils 35 ml 10mM KPi-Puffer (pH 7,0) gespült. Danach werden die Membranproben in 20 ml 10mM KPi-Puffer (pH 7,0) und in 1M wässriger NaCl-Lösung geschüttelt. Die Menge des eluierten Proteins wird durch Messung der optischen Dichte (OD) bei 280 nm bestimmt.
Die Ergebnisse der Bindungskapazität für Lysozym der Proben 1 bis 5 sowie von Sartobind® S sind in Tabelle 2 zusammengefasst.

Beispiel 7: Bindung von Wirtszellproteinen (HCP, "Host-Cell Proteins")

Für die Bestimmung der Bindung von Wirtszellproteinen wird eine 10fach konzentrierte HCP-Lösung in PBS-Puffer (phosphatgepufferte Kochsalzlösung, pH 7,4) ohne Antikörper verwendet, hergestellt bei einem Kontrakthersteller in einem "Mock"-Lauf (Kultivierung einer Zelllinie ohne Antikörperproduktion) einer Ovarienzelllinie chinesischer Hamster. Die HCP-Lösung wird 1:10 in 20mM TRIS/HCl-Puffer (pH 7,4) verdünnt und die Leitfähigkeit wird auf 10 mS/cm durch Zugabe von NaCl eingestellt. 1000 ml der verdünnten HCP-Lösung werden zur Beladung der Membranen verwendet. Die HCP-Konzentration wird mit Hilfe eines ELISA-Tests ("Enzyme-linked immunosorbent assay ELISA Cygnus CM015") nach den Herstellervorgaben bestimmt. Die Konzentration an Wirtszellproteinen (HCP) beträgt 7 µg/ml.
3 Lagen Membran (Probe 4) werden in einen Membranhalter eingespannt. Der Membranstapel weist eine Membranfläche von 15 $cm^2$, eine Anströmfläche von 5 $cm^2$ und eine Betthöhe (Dicke des Membranstapels) von 750 µm in dem Membranhalter auf. Die Membranen in dem Membranhalter werden mit 20mM TRIS/HCl-Puffer (pH 7,4) geflutet, um die Luft zu verdrängen und dann an eine "Äkta® Explorer 100"-Chromatographie-Anlage der Firma GE Healthcare angeschlossen.
Danach werden die Membranen bzw. der Membranstapel mit einem vier Schritte umfassenden Testprogramm hinsichtlich der HCP-Bindung untersucht. Die vier Schritte des Testprogramms sind nachfolgend angegeben:

1. Äquilibrieren der Membran mit 10 ml 20mM TRIS/HCl-Puffer (pH 7,4) mit einer Leitfähigkeit von 10 mS/cm,
2. Beladen der Membran mit 100 ml HCP-Lösung,
3. Waschen mit 10 ml 20mM TRIS / HCl (pH 7,4, Leitfähigkeit 10 mS/cm) und
4. Eluieren mit 10 ml 1M NaCl in 20mM TRIS/HCl-Puffer (pH 7,4).

Alle Schritte werden bei einer Flussgeschwindigkeit von 10 ml/min durchgeführt. Bei allen Schritten wird die Absorption bei 280 nm im Detektor hinter der Membraneinheit gemessen. Die Durchbruchskurven sind in Figur 3 dargestellt.

Beispiel 8: Bestimmung der Bindungskapazität für kleine, inaktivierte Influenzaviren

Influenza A Puerto Rico/8/34, H1N1 wird produziert in adhärenten MDCK-Zellen (GMEM-Medium) (vgl. Y. Genzel et al., Vaccine 22 (2004), 2202-2208). Nach der Kultivierung wird die Kulturbrühe über zwei nacheinander folgende Filtrationsschritte (Porengröße 5 µm und 0,65 µm Tiefenfilter, GE Water & Process Technologies) und anschließend mit 3mM β-Propiolakton für 24 Stunden bei 37 °C chemisch inaktiviert. Nach der Inaktivierung wird die Lösung erneut geklärt (0,45 µm Membranfilter, GE Water & Process Technologies) und anschließend 20-fach aufkonzentriert über Crossflow-Filtration (750 kDa MWCO, GE Healthcare) (vgl. B. Kalbfuss et al., Biotechnol. Bioeng. 97 (1), 2007, 73-85). Die konzentrierten Proben werden bis zur weiteren Verwendung bei -80 °C gelagert.
Die gefrorenen Virus-Aliquote (3 x 2 ml) werden im Wasserbad aufgetaut, gemischt und für 10 min bei 9000 G zentrifugiert. Der Überstand wird anschließend 1:3 mit dem Bindungspuffer (10mM Tris, pH 7,4 und 50mM NaCl) verdünnt.

Die so hergestellte Lösung wird für die Bestimmung der dynamischen Bindungskapazität verwendet. Alle Versuche wurden an einer "Äkta® Explorer 100"-Chromatographie-Anlage der Firma GE Healthcare durchgeführt. Die getesteten Einheiten für die Versuche bestehen aus 15 Lagen Membranen mit einer Anströmfläche von 0,36 $cm^2$ und einem Bettvolumen von 0,14 ml. Die Äquilibrierung erfolgt mit 10mM Tris, 50mM NaCl pH 7,4. Nach der Äquilibrierung wird 26 ml der Virenlösung auf die Einheit beladen und 2 ml Fraktionen werden im Durchlauf gesammelt. Die Flussrate bei der Beladung beträgt 1 ml/min. Die Hämagglutinin-(HA)-Aktivität in den verschiedenen Fraktionen wird quantifiziert. Nach der Beladung werden die Membraneinheiten mit Äquilibrierungspuffer gespült, bis die Basislinie erreicht wird, und anschließend mit 10mM Tris, 2,0M NaCl, pH 7,4 eluiert. Das Eluat und die Spülfraktionen werden auch gesammelt und ebenfalls auf ihre HA-Aktivität analysiert.

In den Durchlauffraktionen wird die Viruskonzentration bestimmt und über den Hämagglutinationsassay quantifiziert (vgl. B. Kalbfuss et al., Biologicals 36 (2008), 145-161). Die Bindungskapazität für Influenzaviren wird für alle Proben gemessen und die Kapazität bei 10 %, 25 % und 50 % Durchbruch, d.h. die Menge an gebundenen Viren pro Volumen Trennmaterial, bis 10% bzw. 25 % oder 50 % der Viruskonzentration der Ausgangslösung erreicht werden, berechnet. Die dynamische Bindungskapazität wurde wie folgt berechnet:

$C_0$: Viruskonzentration in der Ausgangslösung (Viruspartikel/ml = part./ml)
$V_x$: Beladungsvolumen, bis x % Durchbruch erreicht wurden (ml)
$A_x$: Anzahl an Viruspartikeln im Durchlauf, bis x % Durchbruch erreicht wurden
$V_B$: Bettvolumen der getesteten Einheit

Dynamische Bindungskapazität bis x % Durchbruch (part./ml) = $(V_x \times C_0 - A_x)/V_B$

Die Ergebnisse der Bindungskapazitäten der Proben 1 bis 5 sowie für Sartobind® S sind in Tabelle 2 zusammengefasst.

Tabelle 2: Zusammenfassung der Ergebnisse

| Probe | 1 | 2 | 3 | 4 | 5 | Sartobind® S |
|---|---|---|---|---|---|---|
| Porengröße der Cellulose Ausgangsmembran (μm) | 3 | 3 | 3 | 3 | 1,2 | |
| Vernetzung nach | ohne | Beispiel 1 | ohne | Beispiel 1 | Beispiel 1 | nicht zutreffend |
| Sulfatierungsmethode nach | Beispiel 3 | Beispiel 3 | Beispiel 2 | Beispiel 2 | Beispiel 2 | nicht zutreffend |
| Sulfatierungsgrad in Gew.-% | 0,64 | 19,37 | 1,26 | 10,34 | 16,65 | - |
| Bindungskapazität mit Influenza part./ml | | | | | | |
| bis 10 % Durchbruch | 0,0 | $5{,}0 \cdot 10^{11}$ | 0,0 | $3{,}4 \cdot 10^{12}$ | $6{,}4 \cdot 10^{12}$ | $4{,}9 \cdot 10^{11}$ |
| bis 25 % Durchbruch | $43 \cdot 10^{11}$ | $3{,}1 \cdot 10^{12}$ | $4{,}0 \cdot 10^{11}$ | $4{,}3 \cdot 10^{12}$ | nicht erreicht* | $4{,}9 \cdot 10^{11}$ |
| bis 50 % Durchbruch | $4{,}3 \cdot 10^{11}$ | $4{,}4 \cdot 10^{12}$ | $4{,}0 \cdot 10^{11}$ | $5{,}7 \cdot 10^{12}$ | nicht erreicht* | $2{,}3 \cdot 10^{12}$ |
| Bindungskapazität mit Lysozym (mg/mL) | 32,6 | 7,0 | 30,0 | 4,8 | 8,1 | 37,0 |

* Bei der Probe 5 konnte aufgrund der sehr hohen Bindungskapazität dieser Membran für Influenzaviren kein 25-%- bzw. 50-%-Durchbruch mit dem in Beispiel 8 genannten Beladungsvolumen von 26 ml Viruslösung erreicht werden.

## Patentansprüche

1. Sulfatierte Cellulosehydrat-Membran, umfassend eine vernetzte Cellulosehydrat-Matrix mit Poren, die sich von einer Hauptoberfläche zur anderen Hauptoberfläche der Membran erstrecken, wobei die Cellulosehydrat-Membran auf ihren inneren und äußeren Oberflächen Sulfatliganden zur adsorptiven Stofftrennung aufweist, und wobei der Sulfatierungsgrad der Cellulosehydrat-Matrix 15 Gew.-% oder mehr beträgt.

**2.** Sulfatierte Cellulosehydrat-Membran nach Anspruch 1, wobei die mittlere Porengröße der Membran zwischen 0,5 und 5,0 μm beträgt.

**3.** Sulfatierte Cellulosehydrat-Membran nach Anspruch 1 oder 2, wobei der Vernetzungsgrad der Cellulosehydrat-Matrix 0,05 bis 0,5 beträgt.

**4.** Verfahren zur Herstellung einer sulfatierten Cellulosehydrat-Membran nach Anspruch 1, umfassend die Schritte:

- Bereitstellen einer Cellulosehydrat-Membran mit einer Porengröße von 0,1 bis 20 μm;
- Vernetzen der Cellulosehydrat-Matrix mit einem Vernetzungsmittel, welches mindestens zwei funktionelle Gruppen im Molekül aufweist, die mit den Hydroxylgruppen der Cellulosehydrat-Matrix reagieren; und
- Sulfatieren der vernetzten Cellulosehydrat-Matrix.

**5.** Verfahren nach Anspruch 4, wobei das Vernetzungsmittel ausgewählt ist aus der Gruppe, bestehend aus Diepoxidverbindungen, Diisocyanaten, Epichlorhydrin, Epibromhydrin, Dimethylharnstoff, Dimethylethylenharnstoff, Dimethylchlorsilan, Bis-(2-hydroxyethylsulfon), Divinylsulfon, Alkylendihalogenid, Hydroxyalkylendihalogenid und Diglycidylethern oder einem Gemisch daraus.

**6.** Verfahren nach Anspruch 4 oder 5, wobei als Vernetzungsmittel 1,4-Butandioldiglycidylether oder Ethylenglycoldiglycidylether verwendet wird.

**7.** Verfahren nach einem der Ansprüche 4 bis 6, wobei die Konzentration des Vernetzungsmittels in der Vernetzungslösung von 10 bis 30 Gew.-% beträgt.

**8.** Verfahren nach einem der Ansprüche 4 bis 7, wobei die Sulfatierung durch Reaktion der vernetzten Cellulosehydrat-Matrix mit einem Lewisbase-$SO_3$-Komplex erfolgt.

**9.** Verfahren nach einem der Ansprüche 4 bis 8, wobei die Sulfatierung durch Umsetzung mit einem $SO_3$-Pyridin-Komplex erfolgt.

**10.** Verfahren nach Anspruch 9, wobei die Konzentration des $SO_3$-Pyridin-Komplexes in der Sulfatierungslösung 1 bis 40 Gew.-% beträgt.

**11.** Verfahren nach einem der Ansprüche 4 bis 10, wobei die Sulfatierung bei einer Temperatur von 20 bis 90 °C erfolgt.

**12.** Verwendung der sulfatierten Cellulosehydrat-Membran nach einem der Ansprüche 1 bis 3 als Adsorptionsmembran für die Aufreinigung von Viren oder Virusbruchstücken.

**13.** Verwendung nach Anspruch 12 für die Aufreinigung von Viren mit einer Molekülmasse von größer als $10^7$ Da.

**14.** Verwendung nach Anspruch 12 oder 13 für die Aufreinigung von Influenzaviren.

## Claims

**1.** A sulfated cellulose hydrate membrane comprising a crosslinked cellulose hydrate matrix with pores which extend from one main surface to another main surface of the membrane, wherein the cellulose hydrate membrane has sulfate ligands on its inner and outer surfaces for the adsorptive substance separation, and wherein the degree of sulfation of the cellulose hydrate matrix is 15% by weight or more.

**2.** The sulfated cellulose hydrate membrane according to claim 1, wherein the mean pore size of the membrane is between 0.5 and 5.0 μm.

**3.** The sulfated cellulose hydrate membrane according to claim 1 or 2, wherein the degree of crosslinking of the cellulose hydrate matrix is 0.05 to 0.5.

**4.** A method for preparing a sulfated cellulose hydrate membrane according to claim 1, comprising the steps of:

- providing a cellulose hydrate membrane with a pore size of 0.1 to 20 μm;
- crosslinking of the cellulose hydrate matrix using a crosslinker having at least two functional groups in the molecule which react with the hydroxyl groups of the cellulose hydrate matrix; and
- sulfating the crosslinked cellulose hydrate matrix.

5. The method according to claim 4, wherein the crosslinker is selected from the group consisting of diepoxide compounds, diisocyanates, epichlorohydrin, epibromohydrin, dimethylurea, dimethylethyleneurea, dimethylchlorosilane, bis(2-hydroxyethylsulfone), divinylsulfone, alkylene dihalides, hydroxyalkylene dihalides and diglycidyl ethers or a mixture thereof.

6. The method according to claim 4 or 5, wherein 1,4-butanediol diglycidyl ether or ethylene glycol diglycidyl ether is used as crosslinker.

7. The method according to any one of claims 4 to 6, wherein the concentration of the crosslinker in the crosslinking solution is 10 to 30% by weight.

8. The method according to any one of claims 4 to 7, wherein the sulfation is effected by reacting the crosslinked cellulose hydrate matrix with a Lewis base-$SO_3$ complex.

9. The method according to any one of claims 4 to 8, wherein the sulfation is effected by reaction with an $SO_3$-pyridine complex.

10. The method according to claim 9, wherein the concentration of the $SO_3$-pyridine complex in the sulfation solution is 1 to 40% by weight.

11. The method according to any one of claims 4 to 10, wherein the sulfation is effected at a temperature of 20 to 90°C.

12. The use of the sulfated cellulose hydrate membrane according to any one of claims 1 to 3 as adsorption membrane for the purification of viruses or virus fragments.

13. The use according to claim 12 for the purification of viruses having a molecular mass of greater than $10^7$ Da.

14. The use according to claim 12 or 13 for the purification of influenza viruses.

## Revendications

1. Membrane d'hydrate de cellulose sulfatée, comportant une matrice d'hydrate de cellulose réticulée présentant des pores qui s'étendent d'une surface principale de la membrane à l'autre, la membrane d'hydrate de cellulose comportant sur ses surfaces internes et externes des ligands de sulfate pour la séparation de matière par adsorption et le degré de sulfatation de la matrice d'hydrate de cellulose étant de 15 % en poids ou plus.

2. Membrane d'hydrate de cellulose sulfatée selon la revendication 1, dans laquelle la dimension moyenne des pores de la membrane est comprise entre 0,5 et 5,0 μm.

3. Membrane d'hydrate de cellulose sulfatée selon la revendication 1 ou 2, dans laquelle le degré de réticulation de la matrice d'hydrate de cellulose est de 0,05 à 0,5.

4. Procédé pour la fabrication d'une membrane d'hydrate de cellulose sulfatée selon la revendication 1, comprenant les étapes :

- mise à disposition d'une membrane d'hydrate de cellulose ayant une dimension de pores allant de 0,1 à 20 μm ;
- réticulation de la matrice d'hydrate de cellulose à l'aide d'un agent de réticulation qui comporte au moins deux groupements fonctionnels dans la molécule, qui réagissent avec les groupements hydroxyle de la matrice d'hydrate de cellulose ; et
- sulfatation de la matrice d'hydrate de cellulose réticulée.

5. Procédé selon la revendication 4, dans lequel l'agent de réticulation est choisi parmi le groupe constitué par les composés diépoxy, diisocyanates, épichlorhydrine, épibromhydrine, diméthylurée, diméthyléthylène urée, diméthylchlorosilane, bis-(2-hydroxyéthylsulfone), divinyle sulfone, dihalogénure d'alkylène, dihalogénure d'hydroxyalkylène, les éthers diglycidyliques ou des mélanges de ceux-ci.

**6.** Procédé selon la revendication 4 ou 5 dans lequel on utilise l'éther diglycidylique de 1,4-butanediol ou l'éther diglycidylique d'éthylène glycol comme agent de réticulation.

**7.** Procédé selon l'une des revendications 4 à 6 dans lequel la concentration de l'agent de réticulation dans la solution de réticulation est de 10 à 30 % en poids.

**8.** Procédé selon l'une des revendications 4 à 7 dans lequel on effectue la sulfatation par réaction de la matrice d'hydrate de cellulose réticulée avec un complexe de base de Lewis - $SO_3$.

**9.** Procédé selon l'une des revendications 4 à 8 dans lequel on effectue la sulfatation par réaction avec un complexe de $SO_3$-pyridine.

**10.** Procédé selon la revendication 9 dans lequel la concentration du complexe de $SO_3$-pyridine dans la solution de sulfatation est de 1 à 40 % en poids.

**11.** Procédé selon l'une des revendications 4 à 10 dans lequel on effectue la sulfatation à une température de 20 à 90 °C.

**12.** Utilisation de la membrane d'hydrate de cellulose sulfatée selon l'une des revendications 1 à 3 comme membrane d'adsorption pour la purification de virus ou fragments de virus.

**13.** Utilisation selon la revendication 12 pour la purification de virus ayant une masse moléculaire supérieure à $10^7$ Da.

**14.** Utilisation selon la revendication 12 ou 13 pour la purification de virus influenza.

Figur 1

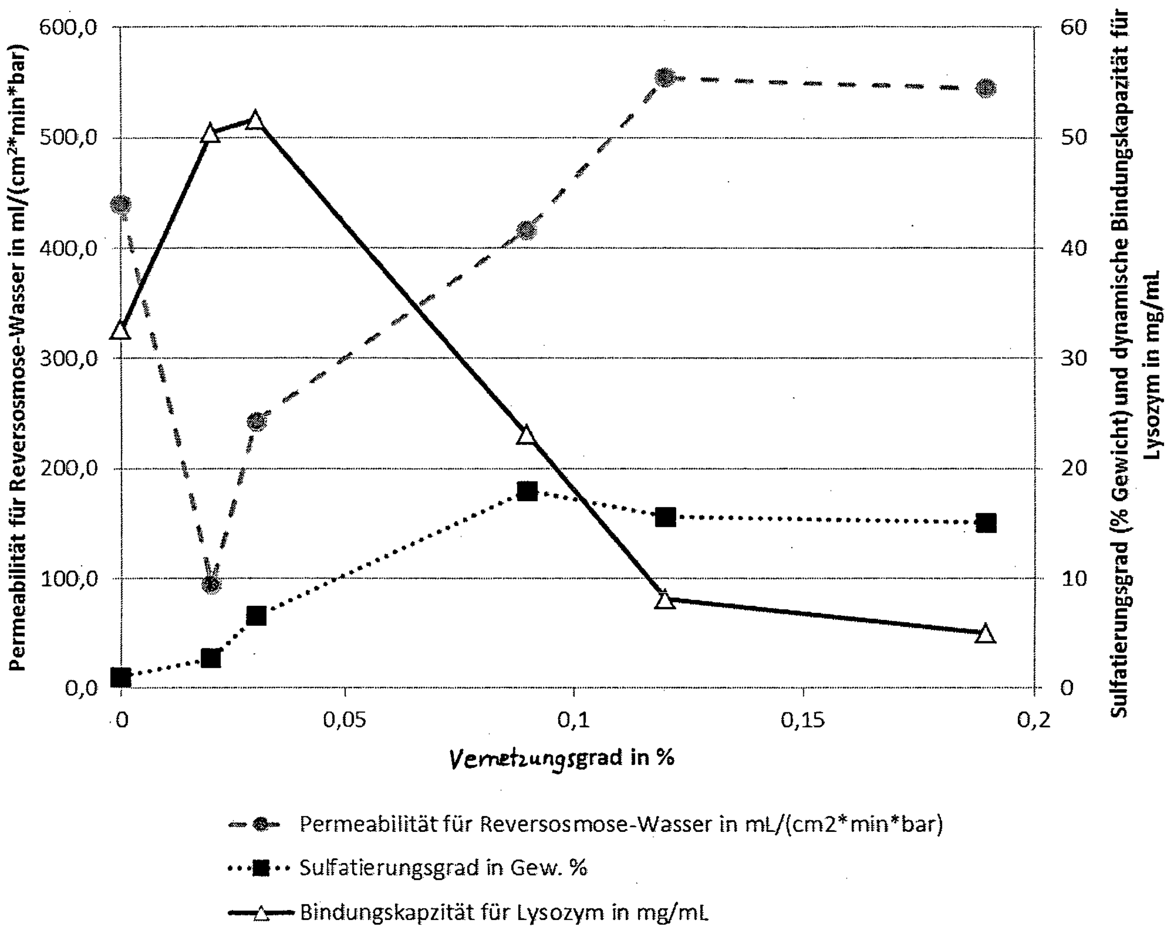
Permeabilität für Reversosmose-Wasser in ml/(cm²*min*bar)
600,0
500,0
400,0
300,0
200,0
100,0
0,0
0
0,05
0,1
0,15
0,2
Vernetzungsgrad in %
60
50
40
30
20
10
0
Sulfatierungsgrad (% Gewicht) und dynamische Bindungskapazität für Lysozym in mg/mL
Permeabilität für Reversosmose-Wasser in mL/(cm2*min*bar)
Sulfatierungsgrad in Gew. %
Bindungskapzität für Lysozym in mg/mL

Figur 2

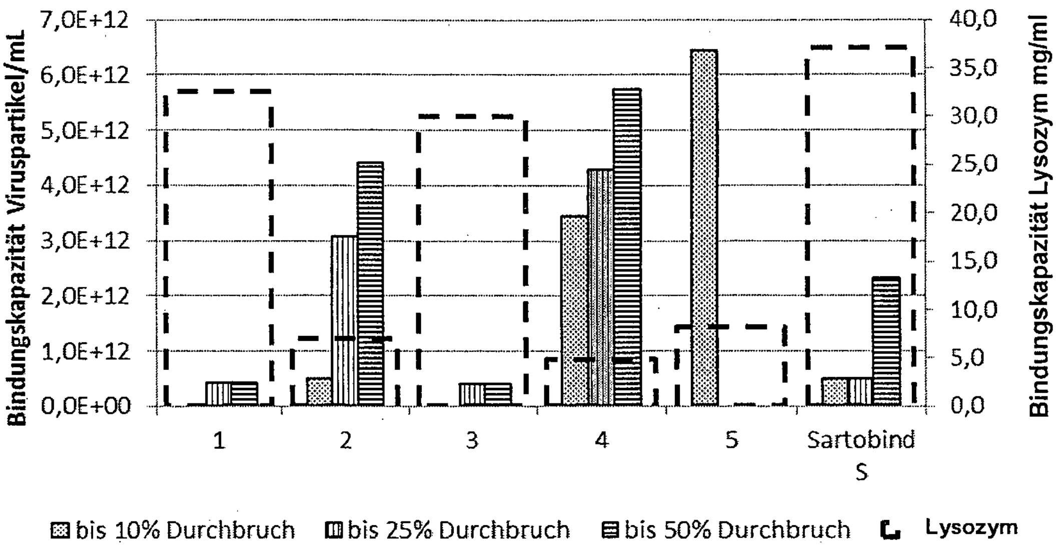
Bindungskapazität Viruspartikel/mL
7,0E+12
6,0E+12
5,0E+12
4,0E+12
3,0E+12
2,0E+12
1,0E+12
0,0E+00
Bindungskapazität Lysozym mg/ml
40,0
35,0
30,0
25,0
20,0
15,0
10,0
5,0
0,0
1
2
3
4
5
Sartobind S
bis 10% Durchbruch
bis 25% Durchbruch
bis 50% Durchbruch
Lysozym

Figur 3

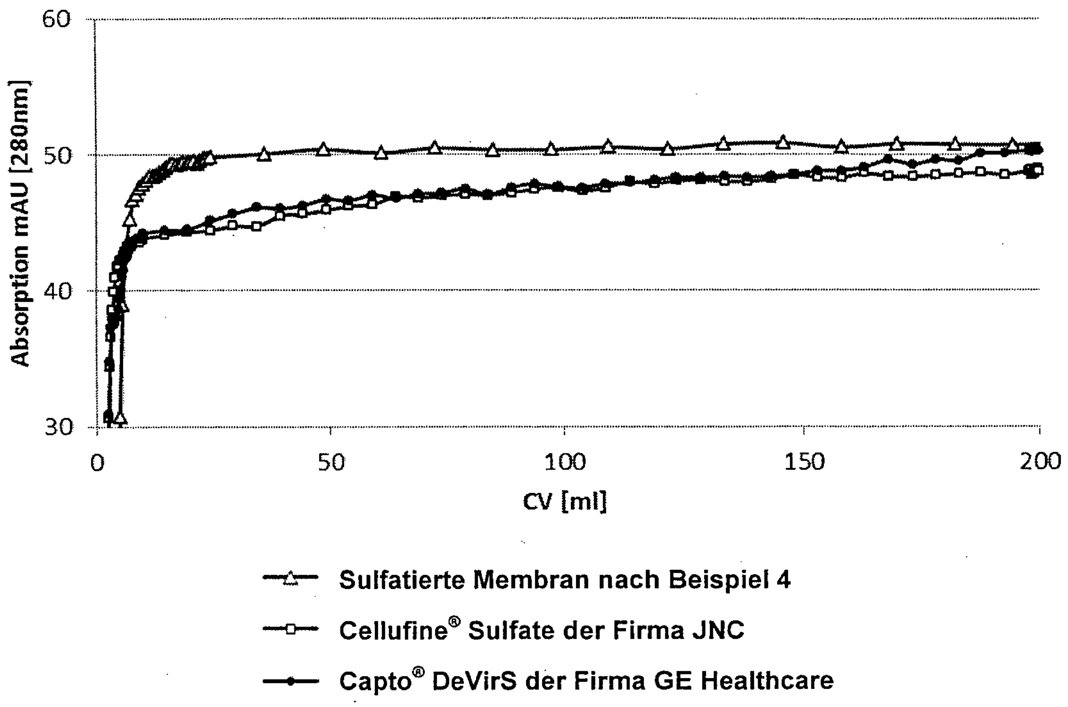
60
50
40
30
Absorption mAU [280nm]
0
50
100
150
200
CV [ml]
Sulfatierte Membran nach Beispiel 4
Cellufine® Sulfate der Firma JNC
Capto® DeVirS der Firma GE Healthcare

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0053473 A1
- EP 1698641 A1
- EP 0171086 A2
- WO 2008039136 A1
- EP 0171771 A2
- EP 0173268 A2
- EP 0171765 A2
- US 5667684 A
- US 8173021 B2
- US 20120171750 A1
- WO 9532793 A1
- DE 10326741 A1
- DE 3447625 A1


### In der Beschreibung aufgeführte Nicht-Patentliteratur


- **R. W. H. RUIGROK et al.** *J. Gen. Virol.,* 1984, vol. 65, 799-802
- **L. OPITZ et al.** *Biotechnology and Bioengineering,* 2009, vol. 103 (6), 1144-1154
- **L. J. ZEMAN et al.** Microfiltration and ultrafiltration principles and applications. Marcel Dekker, 1996
- **Y. GENZEL et al.** *Vaccine,* 2004, vol. 22, 2202-2208
- **B. KALBFUSS et al.** *Biotechnol. Bioeng.,* 2007, vol. 97 (1), 73-85
- **B. KALBFUSS et al.** *Biologicals,* 2008, vol. 36, 145-161